# EUROPEAN PATENT APPLICATION

(11) **EP 1 700 574 A1**
(43) Date of publication of application: **13.09.2006**
(21) Application number: 06002898.2
(22) Date of filing: 14.02.2006
(51) Int. Cl.: A61B 19/00, A61B 17/17

(54) **Apparatus for performing an orthopedic stability test using a surgical navigation system**

(30) Priority: 09.03.2005 US 76084
(71) Applicant: Aesculap AG & Co. KG, 78532 Tuttlingen (DE)
(72) Inventor: Leitner, Jean Francois, 38410 Uriage (FR); Boux de Casson, Francois, 38700 Le Sappey en Chartreuse (FR); Giordano, Nicola, 78056 Villingen-Schwenningen (DE)
(74) Representative: Hoeger, Stellrecht & Partner Patentanwälte

(57) **Abstract**

The present invention provides methods and apparatus that permit one to accurately measure range of motion of a joint for joint stability testing and other purposes. In accordance with one aspect of the invention, a marker that can be tracked by a surgical navigation system is rigidly fixed to each of the bones forming a joint and the joint is placed within the field of view of the surgical navigation system. The surgical navigation system tracks the bones in all six degrees of freedom by tracking the markers attached thereto. The navigation system can be programmed to measure and display to the surgeon all information relevant to such joint stability testing, including, but not limited to, starting position, maximum joint translation, maximum joint rotation, instantaneous joint translation, instantaneous joint rotation, and instantaneous joint flexion angle. The system may be set up to show a comparison of pre- and post-operative data.

## Description

### BACKGROUND

### FIELD OF THE INVENTION

The present invention relates to surgical navigation systems, sometimes called localization devices, and orthopedic stability testing such as knee stability testing.

### BACKGROUND OF THE INVENTION

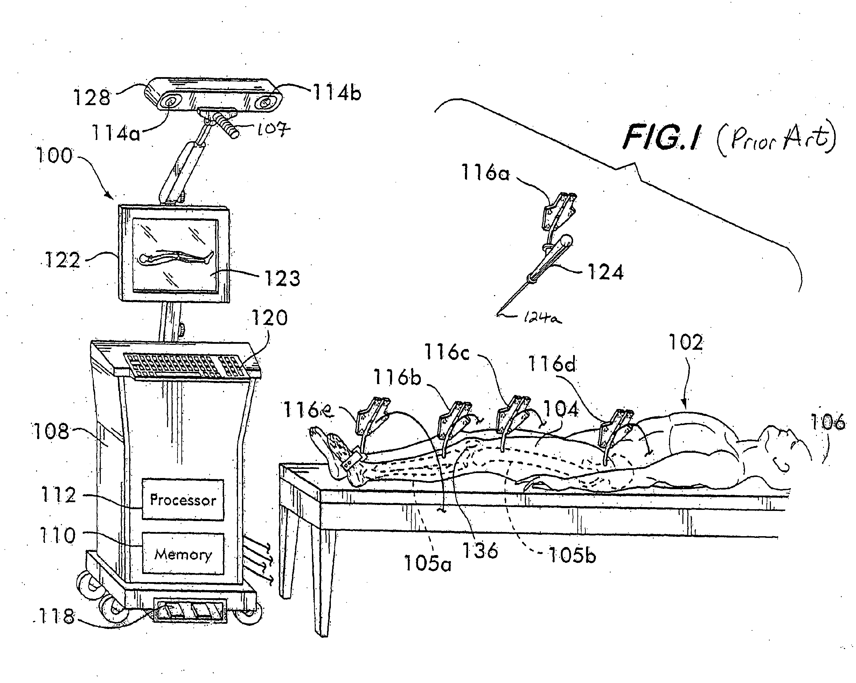

In an exemplary surgical navigation system 100 such as illustrated in Figure 1, at least two sensors 114a, 114b (e.g., infrared cameras) mounted in a housing 128 are used to detect a plurality of markers 116a, 116b, 116c, 116d, 116e that can be mounted on the patient's bones 105a, 105b and/or on surgical tools 124. More particularly, the cameras 114a, 114b are coupled to a computer 112 that analyzes the images obtained by the cameras and detects the positions and orientations of the various bones and/or tools bearing the markers during the surgery and calculates and displays useful information for performing the surgery to the surgeon on a monitor 122. The computer system may be provided in a portable cart 108 and may include a memory 110 for storing data, a keyboard 120, foot pedals 118, and/or other input means for entering data.

One such surgical navigation system is the OrthoPilot available from Aesculap, Inc. of Center Valley, Pennsylvania, USA.

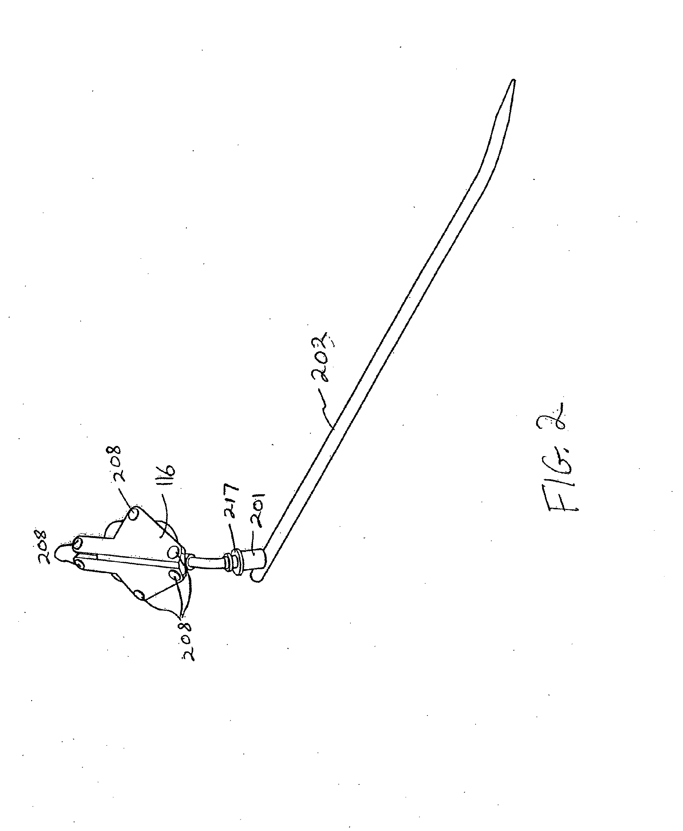

With reference to Figure 2, which is an enlarged view of an exemplary marker 116, which would be fixedly mounted on an anatomical feature (such as a bone) via a bone mounting screw, k-wire, or other attachment mechanism or on a rigid medical instrument (such as a surgical pointer 202). Each marker 116 comprises a base with a mounting mechanism 217 on one end for mounting to a complementary mounting mechanism 201 on the instrument 202. Extending from the other end of the base are at least three infrared LED transmitters 208. Alternately, instead of transmitters, the system could utilize reflective type markers bearing infrared reflectors. When using reflectors, the surgical navigation system includes an infrared light source 107 (Figure 1) directed towards the surgical field so that the reflectors 208 will reflect infrared light back to the two cameras 114a, 114b.

With at least two cameras and at least three transmitters 208 (or reflectors 208a) on a marker, sufficient information is available to the computer to determine the exact position and orientation of each marker 116 in all six degrees of freedom.

In most surgical navigation procedures, it is necessary to discern multiple markers from each other. This can be done in several different ways. If LED transmitters are used, each transmitter 208 can be timed to emit light only during a specific time interval that the computer knows is the time interval assigned to that particular transmitter on that particular marker. The LEDs are illuminated in sequence at a very high rate so that the computer has virtually continuous information as to the exact location of every LED. Alternately, when using reflectors, each marker 116a may have its three or more reflectors 208a positioned in slightly different relative positions to each other so that the computer can discern which marker it is observing by determining the geometric relationship between the three or more reflectors 208a on the marker 116a.

The mounting mechanism at the end of the base of the marker is designed to mate with a complementary mounting mechanism on the surgical instrument in only one position and orientation. The computer is preprogrammed with information relating to the position of the operational portion of the medical instrument relative to the position of the marker when mounted on it. In this manner, by detecting the position and orientation of the marker, the computer will also know the position and orientation of the medical instrument and its operational portion. For instance, the medical instrument may be the pointer 124 shown in Figure 1 having a tip 124a, the exact position of which is known relative to the marker 116a.

Referring back to Figure 1, the markers 116 are fixedly mounted on bones 105 (via bone screws) and or medical instruments 124 (Figure 1) or 202 (Figure 2) positioned within the field of view of the cameras 114a, 114b so that the computer 112 can track the location and orientation of those bones and/or medical instruments. The computer will then generate useful information to help the surgeon determine appropriate locations or alignments for prosthetic implants, cutting jigs, and the like and display it in a display 123 on the monitor 122.

One known use for surgical navigation systems is in knee surgery. In Anterior Cruciate Ligament (ACL) reconstruction surgery, for instance, a surgical navigation system can be used to help define the points on the femur and tibia to which the reconstructed ACL graft must be attached. Particularly, tunnels must be drilled in the femur and the tibia within which the ends of the ACL graft will be affixed. The fixation points on the femur and tibia should be selected so as to meet several criteria, including: (1) that the distance between the fixation point on the femur and the fixation point on the tibia over the entire range of motion of the knee remains as constant as possible so that the ACL is stretched or compressed significantly over the entire range of motion of the knee; (2) a suitable distance between the femoral fixation tunnel point and the over-the-top position, (3) a suitable position of the femoral fixation tunnel point relative to the clock position on the notch surface, and (4) whether the ligament will or will not impinge on the knee surface if fixed at that point.

In ACL reconstruction surgery, two markers 116 may be rigidly fixed to the tibia and femur, respectively, by bone screws or k-wires. A third marker may be attached to a surgical pointer. While tracking the markers on the tibia, femur, and pointer, respectively, the pointer is used to palpate a series of points on the tibia and femur from which information defining the best fixation points for the ACL graft can be calculated. The surgical navigation system records the points palpated on the tibia in memory as a function of the position of the tibial marker and records the points palpated on the femur in memory as a function of the position of the femoral marker. As part of this process, the surgeon is prompted to palpate with the pointer a plurality of points on the notch surface of the femur near the expected fixation point of the ACL graft on the femur. Eventually, the surgeon is asked to flex and rotate the knee joint while the surgical navigation system records the motion of the femoral and tibial markers relative to each other. When enough points have been gathered, all of the aforementioned collected information can then be used to determine the best position to drill the tunnels in which the ACL graft will be fixed to the tibia and femur, respectively, as described below

In the navigation phase of the procedure, the surgeon first mounts another marker to a tibial drill guide and navigates it to the appropriate position using various criteria displayed on the monitor of the surgical navigation system and drills the tibial fixation tunnel. The surgical navigation system then calculates an "isometry" value for each of a plurality of palpated potential femoral fixation points relative to the tibial fixation tunnel. The isometry values essentially are the maximum change in distance between the tibial fixation tunnel and the potential femoral fixation points over the range of motion of the knee joint. A marker is then mounted to a femoral drill guide and the surgical navigation system tracks the femoral drill guide while the monitor shows the surgeon several different criteria as a function of the position of the drill guide relative to the femur that are useful for determining the ideal placement of the femoral fixation point (i.e., the femoral drill guide). One of those values is the isometry value, however, other criteria include (1) the distance between the femoral fixation tunnel point and the over-the-top position, (2) the position of the femoral fixation tunnel point relative to the clock position on the notch surface, and (3) whether the ligament will or will not impinge on the knee surface if fixed at that point.

The surgeon navigates the femoral drill guide on the femur until he locates a mounting location that is the best compromise between all of the criteria and attaches the drill guide and drills the femoral fixation tunnel. The remainder of the surgical procedure is then performed in the usual fashion.

One common test used by surgeons to determine how well a reconstructed ligament will function, such as a reconstructed ACL, is to measure the maximum range of motion of the joint in one or more degrees of freedom after the graft ligament has been installed. In fact, it is often very useful to measure the difference in range of motion in certain degrees of freedom of the joint before and after the surgery.

For instance, in ACL reconstruction surgery, two motions are of particular interest. They are the full range of translation of the tibia relative to the femur along a straight line orthogonal to the tibia when the knee is bent to approximately 30° flexion and/or 90° flexion and the full range of rotation of the tibia relative to the femur about the mechanical axis of the tibia.

Particularly, generally, the ranges of translation as well as rotation are greater in the case of a damaged ACL relative to a healthy ACL. Thus, it is common to measure the success of an ACL reconstruction surgical procedure by comparing the pre and post-operative translational and rotational ranges of the knee joint with the goal being smaller ranges of motion after the surgery than before the surgery.

such tests also may be performed to determine if the patient's ligament has suffered injury at all. In this type of situation, one might compare the ranges of motion of the patient's healthy knee with the same ranges for the patient's potentially injured knee. Alternately, the ranges of motion may be compared to charts disclosing the normal ranges of motion for a comparable healthy knee.

Such range of motion (or stability) tests normally are performed manually by the surgeon gripping the patient's tibia with both hands are pushing and pulling on it in the anterior/posterior direction and/or rotating it about its mechanical axis. This procedure often is performed in a clinical setting prior to the surgery and after the surgery and the results are compared to determine how well the reconstructed ligament will work. However, it also can be performed in the surgery theater during the surgery, particularly, the "after" test.

The translational test is commonly known as the Lachmann test or the anterior-drawer test, the difference between the two being the flexion angle of the knee during the test. The rotational test is commonly called a medial/lateral rotation test.

The Lachmann and medial/lateral rotation tests usually are performed without measuring instruments whereby the surgeon simply uses his innate sense of feel to determine the before and after differences in motion. Sometimes a measuring instrument is attached to the patient's calf and provides a more precise measurement of the range of translational motion during the Lachmann or anterior drawer test. These instruments are known by various different names, including Rolimeter™, available from Aircast Inc., and Arthrometer®, available from MEDmetric Corp.

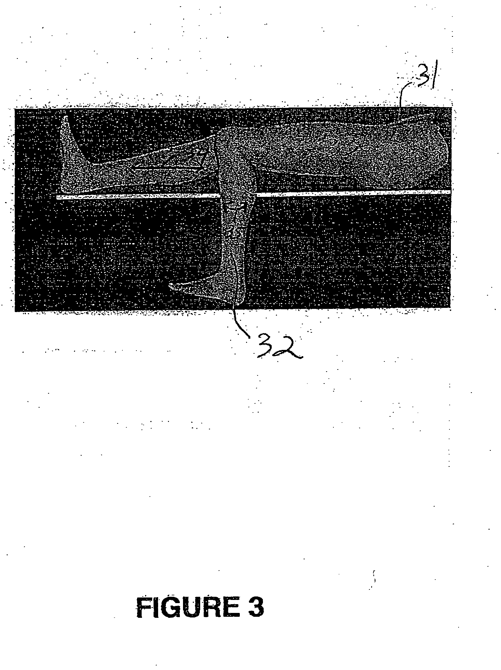

Figure 3 illustrates the two types of motions involved in the aforementioned joint stability tests. The patient's leg 31 is shown with the calf portion 32 at a flexion angle of about 90° relative to the thigh portion 33. This is the desired flexion angle for the anterior-drawer test. The translational movement for this test involves pushing and pulling the calf in the direction of the axis 34 perpendicular to the axis of the calf and generally in the sagittal plane without rotating the calf orientation (i.e., translation). The Lachmann test is essentially identical, but with the knee bent to 30° flexion, rather than 90° flexion. For the medial/lateral rotation joint stability test, the range of motion being determined is illustrated by item 35 in Figure 3. It is a rotation about the mechanical axis of the calf (i.e., of the tibia). For the medial/lateral rotation joint stability test, the knee would normally be bent to 30° flexion; however, for sake of economy of drawings, it is illustrated in the same Figure 3 (with the knee bent to 90° flexion).

It is an object of the present invention to provide an improved method and apparatus for surgical navigation.

It is another object of the present invention to provide an improved method and apparatus for measuring joint stability.

### SUMMARY OF THE INVENTION

The present invention provides methods and apparatus that overcome the aforementioned problems by permitting one to more accurately and easily measure range of motion of a joint for joint stability testing or other purposes. In accordance with one aspect of the invention, markers that can be tracked by a surgical navigation system are rigidly fixed to each of the bones forming a joint and the joint is placed within the field of view of a surgical navigation system. The surgical navigation system tracks the bones in all six degrees of freedom by tracking the markers attached thereto. The navigation system can be programmed to measure and display to the surgeon any and all information relevant to such joint stability testing, including, but not limited to, starting position, maximum joint translation, maximum joint rotation, instantaneous joint translation, instantaneous joint rotation, and instantaneous joint flexion angle. The system may be set up to show a comparison of pre- and post-operative data, such as pre- and post-operative maximum rotation and translation measurements. The system also may be set up to issue a warning signal if the test conditions for two measurements that are to be compared (e.g., pre- and post-operative or good and injured joint) differ by greater than a predetermined threshold. These conditions for instance may comprise the flexion angle of the joint during the test, or the speed of bone movement during the test.

The system also can be programmed to guide the surgeon through the steps of the various tests, such as by providing on-screen instructions for each step of the test procedure.

### BRIEF DESCRIPTION OF THE DRAWINGS

Figure 1 is an illustration of a surgical navigation system being used in connection with knee surgery in accordance with the prior art.

Figure 2 is a close-up perspective view of a marker of the LED emitter type for use with a surgical navigation system mounted on a surgical pointer in accordance with the prior art.

Figure 3 is a drawing illustrating the motions associated with the anterior-drawer type joint stability test, Lachmann joint stability test, and the medial/lateral rotation type joint stability test on a knee joint.

Figure 4 is a drawing illustrating a vector measurement of maximum range of motion in accordance with one embodiment of the present invention.

Figure 5 is an illustration of a screen displayed on the monitor of the surgical navigation system for collecting pre-reconstruction joint stability data.

Figure 6 is an illustration of a screen displayed on the monitor of the surgical navigation system for collecting post-reconstruction joint stability data.

### DETAILED DESCRIPTION OF THE INVENTION

A description of a suitable surgical navigation system (also sometimes called a localization device) for use in connection with the present invention is found in U.S. Patent No. 6,385,475 to Cinquin et al., incorporated herein by reference.

In accordance with the present invention, a marker that can be tracked in all six degrees of freedom (X, Y, Z, rotation about the X axis, rotation about the Y axis, and rotation about the Z axis) by a surgical navigation system is rigidly attached to each bone forming a joint. For instance, if the joint of interest is the knee joint, then one marker would be attached to the tibia and the other marker would be attached to the femur. If one of the bones can be held relatively steady, it may not be necessary to track both bones for purposes of the present invention. Mechanisms are available, for instance, for holding a patient's thigh (or other limb) relatively immobile for purposes of medical procedures and are often used in medical techniques involving surgical navigation. See, for instance, U.S. Patent No. 5,611,353, which discloses one particular medical procedure for determining the mechanical axis of a patient's leg using a surgical navigation system and employing a support arm for fixedly positioning a femur.

In order to accurately measure translation of the tibia relative to the femur for purposes of a Lachmann test and/or anterior-drawer test of joint stability and/or in order to accurately measure rotation of the tibia about its mechanical axis for purposes of a medial-lateral rotation test of joint stability, no kinematic data about the knee need be obtained. That is, the translation of the tibia relative to the femur as well as the rotation of the tibia about its longitudinal axis relative to the femur can be measured by the surgical navigation system merely by tracking the two markers without the need for any other information about the bones. However, as previously noted, it is important to conduct such tests at a particular flexion angle of the joint. For instance, for the Lachmann and anterior-drawer tests, the knee should be bent to approximately 30° flexion. For the anterior-drawer test, the knee should be bent at approximately 90° flexion.

In addition, if the surgeon wishes to collect joint stability data for purposes of comparing it to comparable joint stability data such as before and after comparisons are right knee/left knee comparisons, it is important to assure that the two sets of data that are being compared have been performed at approximately the same flexion angle so that the comparison is most meaningful.

Therefore, it is desirable to also measure the flexion/extension angle of the joint and display it to the surgeon so that the surgeon can assure he/she is performing the tasks at the desired flexion/extension angle. If it is desired to measure the flexion/extension angle of the joint, it will be necessary to obtain additional kinematic data about the joint from which the mechanical axis of each of the tibia and the femur can be calculated so that changes in the mechanical axes of the tibia and the femur can be tracked based on the positions of the tibial and femoral markers, respectively. Aforementioned U.S. Patent No. 6,385,475 to Cinquin et al. discloses a technique for kinematically determining the mechanical axes of the bones forming a joint using a surgical navigation system. Once the mechanical axes of the femur and tibia are known, then the flexion/extension angle of the joint is known from the relative orientations of the tibial and femoral markers.

In accordance with the invention, after the markers have been mounted and, optionally, after the mechanical axes of the bones have been determined as a function of the marker orientations, the surgeon performs a Lachmann test, anterior-drawer test, and/or a medial/lateral rotation test of the joint largely in the normal fashion. Particularly, for the Lachmann test, the surgeon would bend the knee to about 30° flexion and then grasp the patient's calf with both hands close to the knee, i.e., near the back of the knee, and then pull the calf forward in a direction generally perpendicular to the mechanical axis of the tibia with a predetermined amount of force until the calf will not move forward any further. Typically, the surgeon will do this several times to relax the muscles and also to assure that the measurements are repeatable. In one embodiment of the invention, the surgeon could employ an instrument such as a Rolimeter™ or Arthrometer®, that measures the force as it is being applied so that the surgeon can more accurately and repeatably apply the proper amount of force. On the other hand, it may be difficult or impossible to mount such instruments on the patient's calf when the knee is surgically opened.

The anterior-drawer test is essentially identical to the Lachmann test except that the knee is bent to approximately 90° flexion instead of approximately 30° flexion.

The system can measure the maximum translation of the joint in one of two ways. In the first technique, the maximum measurement could is calculated as the largest straight line distance between any two detected positions of the positions of the markers.

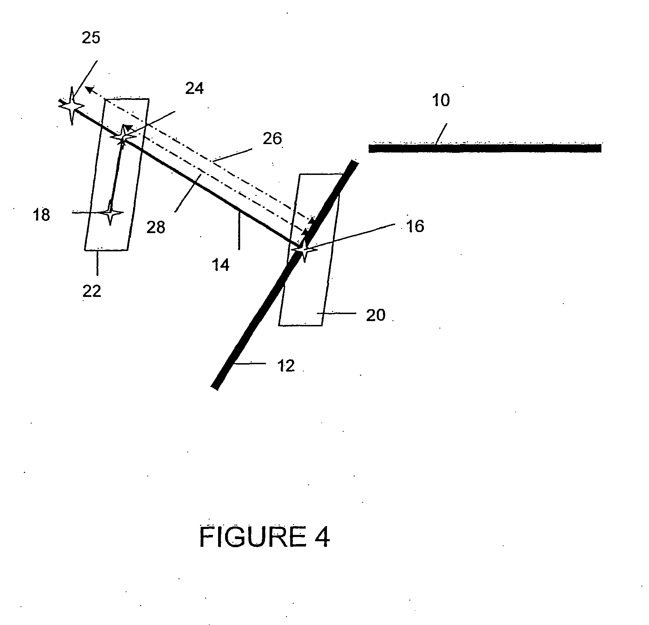

In the second technique, the maximum measurement is calculated as the maximum translation along a predetermined path, i.e., the maximum distance between any two detected positions of the markers measured along a predetermined vector, such as a vector running in the anterior/posterior direction or a line in the sagittal plane and perpendicular to the mechanical axis of the tibia. Stated another way, the maximum translation is the maximum distance between any two parallel planes defined by the marker positions measured along a straight line perpendicular the those planes, such as illustrated in Figure 4.

Referring to Figure 4, the mechanical axis of the femur is represented by line 10 and the mechanical axis of the tibia is represented by line 12. The desired translational vectoral path for the tibia for purposes of a Lachmann test is perpendicular to the mechanical axis of the tibia and in the sagittal plane, as shown by vector 14. Let us assume that the starting point of the tibial marker is point 16. Point 16 lies in one particular plane 20 that is perpendicular to vector 16. If the individual moving the patient's tibia translates the patient's leg perfectly in the direction of vector 14, then, at maximum translation, the point 25 represented by the marker at maximum translation will lie on vector 16. Thus, the maximum translation is simply the distance 26 along vector 14 between point 16 and point 25. However, if the individual performing the test does not translate the patient's tibia perfectly in the direction of vector 14, the point represented by the marker will not end up on vector 14, but on some point off of vector 14, such as point 18. Point 18 defines a single plane that is perpendicular to plane 20, namely plane 22. Thus, in order to calculate the maximum translation, each point observed by the system that not on vector 14 is projected onto vector 14 in a plane parallel to plane 20. For instance, point18 would be projected in plane 22 onto vector 14, resulting in point 24, having a distance 28 from point 16. It is the distance from point 16 to the maximum one of these projected marker points that is taken as the maximum translation value of the tibia.

The second technique more accurately reflects the measurement desired in the Lachmann and anterior-drawer tests because it somewhat compensates for the fact that the individual performing the test will not always translate the patient's tibia exactly along the desired vector.

For the medial/lateral rotation test, the knee should be bent to approximately 30° flexion. The surgeon then grasps the patient's calf with both hands and twists it about the mechanical axis of the tibia with a predetermined amount of force to the point where the resistance to the rotation prevents further rotation. This is repeated several times for both medial rotation and lateral rotation.

In accordance with the present invention, the surgical navigation system tracks the tibial and femoral markers during the tests and records in memory and displays on the monitor useful test data such as the flexion angle of the knee joint, the maximum translation, and the maximum rotation. When the test is the latter of two tests being performed for purposes of comparison, the system should display comparative information from the earlier test while simultaneously displaying the data from the present test being performed.

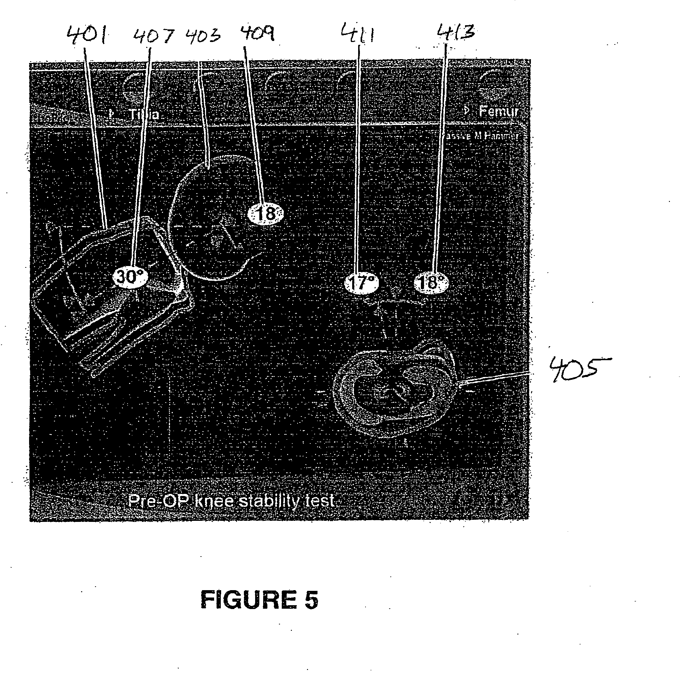

Figure 5 illustrates an exemplary display for use in recording and displaying knee stability testing data in accordance with one embodiment of the present invention. In this embodiment a sagittal outline drawing 401 of the top of the tibia and a sagittal outline drawing 403 of the bottom of the femur are shown in order to provide a visual reference to the surgeon. In a preferred embodiment of the invention, the outline drawings are standardized representations and are not representative of the actual shape of the patient's bones, but only of their relative positions to each other as discussed in detail below.

Particularly, as the patient's bones are moved relative to each other, the outline drawings of the tibia and femur on the monitor move in synchronicity with the actual bone movement in order to provide the surgeon with visual cues and assurance that the bone motion is being properly tracked by the system. Thus, for instance, as the surgeon changes the flexion angle of the patient's knee, the tibia representation 401 will rotate about the femur representation 403 on the monitor the same amount as the actual bones are rotating. Likewise, as the surgeon translates the tibia relative to the femur during the Lachmann and/or anterior-drawer test, the display on the monitor will show the same translational movement of the two bones relative to each other.

In addition, a top plan view 405 of the tibia is shown to the right of the sagittal view. Also, an oval 407 is shown within which appears a numerical representation of the flexion angle of the knee joint. Another oval 409 shows the translational displacement of the tibia relative to the femur. In one embodiment, oval 409 is configured to show the maximum translation from the rest position that occurred during the particular test. However, in other embodiments, the oval can show the instantaneous translational displacement. This may be displayed instead of or in addition to the maximum displacement. In another embodiment, both the instantaneous and maximum values may be displayed simultaneously in different ovals or in the same oval, such as with the maximum value appearing above the instantaneous value. The right hand display 405 is used to display to the surgeon the medial/lateral rotation test data. Particularly, as the surgeon rotates the patient's calf, the outline drawing 405 rotates on the screen correspondingly. In addition, ovals 411 and 413, respectively, show the medial and lateral rotational angles of the tibia relative to the femur. As in the case of the Lachmann test, the ovals preferably show the maximum medial and lateral rotation values, respectively. However, in other embodiments of the invention, they may show the instantaneous rotational values either instead of or in addition to the maximum rotational values. The information that the surgeon is primarily interested in in connection with these tests is the maximum values. Accordingly, even if not displayed in this particular screen, the surgical navigation system should record the maximum values and make them available for viewing by the surgeon at some point.

The surgeon must input data to the surgical navigation system so that the system knows when the test begins and ends. In one embodiment, the surgeon initiates the knee stability data gathering program by activating an input device such as a foot pedal, mouse, or key. Such activation calls up the screen illustrated in Figure 5. The surgeon would then bend the patient's knee to the desired flexion angle, e.g., 30°, and then perform the test.

In a preferred embodiment of the invention, the surgical navigation system is programmed with the desired flexion angle of the joint for purposes of the given test. Alternately, the surgeon may be allowed to set it manually at the time of the test by, for instance, rotating the knee to the desired flexion angle and then depressing a key or foot pedal to indicate to the system that this is the angle at which he wishes to perform the test. In such an embodiment, the system might be programmed to warn the surgeon if and when he/she inadvertently rotates the joint too far away from the desired flexion angle. For instance, in one embodiment of the invention, the system can issue an audible or visual warning when the instantaneous flexion angle is more than ±5° from the preset desired angle. Alternatively, the oval 407 displaying the flexion angle can be caused to disappear (or the oval can remain and just the number within the oval can be caused to disappear) if the angle varies from the preset angle by more than 5 degrees. In one preferred embodiment, the number displayed in oval 407 changes color, for instance, from green to red, when the flexion angle is more than 5° from the preset angle.

When the test is completed, the surgeon again should indicate this to the system via an input device. In one embodiment of the invention, this may be achieved by depressing a particular foot pedal while the screen shown in Figure 5 is displayed on the monitor. In one preferred embodiment of the invention, the system is programmed to detect the difference between a quick press and release of the foot pedal and a long press and release of the foot pedal so that a single foot pedal can be used to signify two different things to the system. Of course, the significance of any given pedal press also is a function of the particular screen that is being displayed when the pedal is pressed. In this manner, a single foot pedal can be used to enter all data, because the system is aware of which data is being entered at any given time by the type of pedal press (e.g., quick or long) and the particular screen that is being displayed at the time.

The functionality of a single input device, such as a foot pedal can further be extended by programming the system to recognize even further activation sequences, such as a quick double press and release or by distinguishing between a slow depression versus a quick depression of the foot pedal.

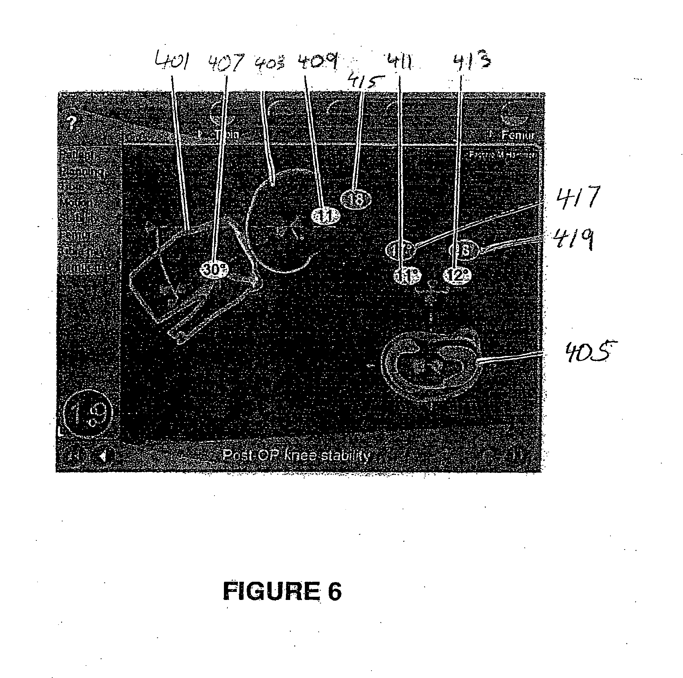

As previously noted, one of the most prevalent uses of the joint stability tests is for the purpose of comparing the results to other results, such as results for the same tests performed on the other knee or results from before and after a medical procedure such as an ACL reconstruction. Accordingly, Figure 6 is a screen shot of an exemplary display for use in such comparative circumstances. For instance, the surgeon might be presented with the display of Figure 6 for a post-operative joint stability test after he/she has performed a pre-operative knee stability test. Figure 6 is very similar to Figure 5 except for the addition of several more ovals. Particularly, outline drawing of the tibia 401, outline drawing of the femur 403, plan view drawing of the tibia 409, and ovals, 407, 409, 411, and 413 are essentially the same as in Figure 5. However, in addition, immediately adjacent oval 409 is a second oval 415 showing the maximum translation value from the earlier test to which the currently collected data is to be compared. For instance, oval 415 shows the maximum translation from the pre-operative test illustrated by Figure 5. Likewise, ovals 417 and 419 are shown immediately adjacent ovals 407 and 409, respectively, and show the maximum medial and lateral rotation values collected during the pre-operative knee stability test.

Although the system can and does track the femur in the described embodiment of the invention, and, therefore, can provide accurate measurements even if the femur moved his during any of the testing, it is preferable to move the femur as little as possible during such testing. If the femur moves too much, the data may be unreliable even though it is being tracked. Therefore, in a preferred embodiment of the invention, the surgical navigation system monitors the movement of the femur and, if it exceeds a predetermined threshold, it warns the surgeon that the data has become unreliable. Hence, the surgeon can restart the test. In a preferred embodiment of the invention, the system will automatically restart the data collection after issuing such a warning. The warning may take the form of a written warning posted on the screen.

While the invention has been described in connection with a surgical theater environment in which the markers are rigidly attached directly to the bones after the bone has been surgically exposed, the invention also may be employed in a clinical setting wherein the markers are attached to the skin of the patient's thigh and calf. Obviously, in such applications, the measurements will not be as accurate as when the markers can be mounted directly to the bones since the skin can move relative to the bones during the tests resulting in inaccuracies. Nevertheless, the technique still is substantially more accurate that the aforementioned former techniques for measuring the ranges of motion for these tests.

Furthermore, while the invention has been particularly described in connection with ACL reconstruction surgery, it can be applied in connection with any ligament and any joint.

Having thus described a few particular embodiments of the invention, various alterations, modifications, and improvements will readily occur to those skilled in the art. Such alterations, modifications and improvements as are made obvious by this disclosure are intended to be part of this description though not expressly stated herein, and are intended to be within the spirit and scope of the invention. Accordingly, the foregoing description is by way of example only, and not limiting. The invention is limited only as defined in the following claims and equivalents thereto.

## Claims

1. A method of using a surgical navigation system for measuring range of motion of a joint, said method comprising the steps of:
(1) fixedly mounting a first marker trackable by said surgical navigation system to a first bone forming said joint;
(2) fixedly mounting a second marker trackable by said surgical navigation system to a second bone forming said joint;
(3) moving said first bone relative to said second bone along a predetermined path while said surgical navigation system tracks said first and second markers; and
(4) storing in a memory of said surgical navigation system a maximum displacement along said path of said second bone relative to said first bone

2. The method of claim 1 further comprising the step of:
(5) displaying said maximum displacement.

3. The method of claim 2 wherein step (3) comprises translating said first bone relative to said second bone in a direction perpendicular to the first bone.

4. The method of claim 2 wherein step (3) comprises rotating said first bone relative to said second bone around a longitudinal axis of said first bone.

5. The method of claim 3 further comprising the steps of:
(6) kinematically determining the mechanical axis of said first and second bones; and
(7) tracking the flexion angle of the joint.

6. The method of claim 5 further comprising the steps of:
(8) setting a predetermined flexion angle for said joint for purposes of taking said measurement; and
(9) displaying the instantaneous flexion angle of said joint during step (3).

7. The method of claim 6 further comprising the step of:
(10) generating an indication when said instantaneous flexion angle of said joint differs from said predetermined flexion angle by a predetermined amount.

8. The method of claim 2 further comprising the step of:
(11) determining if the second bone moves a predetermined amount during step (3); and
(12) if said second bone moves greater than said predetermined amount during step (3), generating an indication of such condition.

9. The method of claim 2 wherein step (3) comprises performing a Lachmann test.

10. The method of claim 2 wherein step (3) comprises performing an anterior-drawer test.

11. The method of claim 2 wherein step (3) comprises performing a medial/lateral rotation test.

12. The method of claim 2 wherein step (3) comprises tracking said markers in six degrees of freedom.

13. The method of claim 2 wherein step (5) comprises further displaying a reference value for purposes of comparison with said displayed maximum value.

14. The method of claim 13 wherein said reference value is a value for said maximum displacement from a previous iteration of said method.

15. The method of claim 14 wherein said previous iteration of said method was performed prior to a surgical procedure and the present iteration of said method is performed after said surgical procedure.

16. The method of claim 14 wherein said previous iteration of said method was performed in connection with a corresponding healthy joint of said patient.

17. An surgical navigation apparatus for performing range of motion tests on an anatomical joint comprising:
a first marker for fixedly mounting to a first bone forming an anatomical joint;
a second marker for fixedly mounting to a second bone forming said anatomical joint;
sensors for tracking said first and second markers;
a monitor for displaying data of said range of motion of said anatomical joint; and
a computer coupled to said sensors and said monitor for tracking said first and second markers, said computer adapted to track motion of said first marker relative to said second marker and to store and cause said monitor to display data of said range of motion along said predetermined path.

18. The apparatus of claim 17 wherein said displayed data comprises a maximum range of motion along a straight line path.

19. The apparatus of claim 17 wherein said displayed data is a maximum range of rotation of said second bone about a longitudinal axis of said second bone.

20. The apparatus of claim 17 wherein said computer is further adapted to kinematically determine the mechanical axis of said first and second bones and track the flexion angle of the joint.

21. The apparatus of claim 20 wherein said computer is further adapted to determine and display an instantaneous flexion angle of said joint.

22. The apparatus of claim 21 wherein said computer is further adapted to generate an indication when said instantaneous flexion angle of said joint differs from a predetermined flexion angle of said joint by a predetermined amount.

23. The apparatus of claim 17 wherein said computer is further adapted to determine if said second bone moves a predetermined amount during said test and, if said second bone moves greater than said predetermined amount during said test, generate an indication of such condition.

24. The apparatus of claim 17 wherein said computer is adapted to track said first and second markers in six degrees of freedom.

25. The apparatus of claim 24 wherein said markers comprise rigid bodies, each bearing at least three trackable elements in fixed spatial relation to each other and said apparatus further comprises at least two sensors.

26. The apparatus of claim 25 wherein said trackable elements comprise electromagnetic emitters.

27. The apparatus of claim 24 wherein said trackable elements comprise infrared reflectors and said apparatus further comprises a source of infrared radiation.

28. The apparatus of claim 27 wherein said computer is further adapted to display a reference value for purposes of comparison with said displayed maximum value.

29. The apparatus of claim 28 wherein said reference value is a previously measured value for said maximum displacement.

30. An surgical navigation apparatus for performing range of motion tests on an anatomical joint comprising:
a marker for fixedly mounting to a first bone forming an anatomical joint;
sensors for tracking said marker;
a monitor for displaying data of said range of motion of said anatomical joint; and
a computer coupled to said sensors and said monitor for tracking said marker, said computer adapted to track motion of said marker and to store and cause said monitor to display data of said range of motion along said predetermined path.

31. The apparatus of claim 30 wherein said displayed data comprises a maximum range of motion along a path.

32. The apparatus of claim 30 further comprising;
means for fixedly holding a second bone forming said anatomical joint.

33. The apparatus of claim 30 wherein said computer is further adapted to kinematically determine the mechanical axis of said first and second bones and track the flexion angle of the joint.

34. The apparatus of claim 33 wherein said computer is further adapted to determine and display an instantaneous flexion angle of said joint.

35. The apparatus of claim 34 wherein said computer is further adapted to generate an indication when said instantaneous flexion angle of said joint differs from a predetermined flexion angle of said joint by a predetermined amount.

36. The apparatus of claim 30 wherein said computer is further adapted to display a reference value for purposes of comparison with said displayed maximum value.

37. The apparatus of claim 36 wherein said reference value is a previously measured value for said maximum displacement.
